# EUROPEAN PATENT SPECIFICATION

(11) **EP 0 413 152 B1**
(45) Date of publication and mention of the grant of the patent: **10.01.1996**
(21) Application number: 90113663.0
(22) Date of filing: 17.07.1990
(51) Int. Cl.: C07D 311/06

(54) **7-Hydroxy coumarins having substitutions in the 4 position**
7-Hydroxy-Cumarine mit Substituenten in 4-Stellung
7-Hydroxy coumarins avec substitutions en position 4

(30) Priority: 17.08.1989 US 394052
(43) Date of publication of application: 20.02.1991
(73) Proprietor: ABBOTT LABORATORIES, Abbott Park, Illinois 60064-3500 (US)
(72) Inventor: Bouma, Stanley Robert, Mundelein, Illinois 60060 (US); Celebuski, Joseph E., Gurnee, Illinois 60031 (US)
(74) Representative: Modiano, Guido, Dr.-Ing.

(56) References cited:
- EP-A- 0 175 541
- DE-A- 3 243 158
- US-A- 4 618 622
- CHEMICAL ABSTRACTS, vol. 102, no. 11, March 18, 1985, Columbus, Ohio, US; PAL R.; PETRI W.A., JR.; BEN-YASHAR, V.; WAGNER, R.R.; BARENHOLZ, Y. "Characterization of the fluorophore 4-heptadecyl-7-hydroxycoumarin: a probe for the head-group region of lipid bilayers and biological membranes.", page 281, column 2, abstract-no. 92 358y

## Description

### Background of the Invention

This invention relates to fluorescent 7-hydroxy coumarin compounds with substitutions in the 4 position having a length greater than one carbon atom. The compounds thus are derivatives of 4-methylumbelliferone (7-hydroxy-4-methyl coumarin, or 4-MU), the detectable label used in the IMₓ® instrument assays (Abbott Laboratories, Abbott Park, IL).

A number of fluorometric labels are known to one of ordinary skill in the art. However, for compatibility reasons, applicants desired a fluorophore label that had electronic properties substantially similar to the 4-MU utilized in the IMₓ® instrument. Otherwise, the label might fluoresce at a wavelength the instrument could not detect absent special filters and the like. A label optimized to the existing instrument was necessary.

The search began for a coumarin or umbelliferone nucleus that had an activated or activatable tether that could be coupled to a desired molecule. Such a tether to a coumarin nucleus had been obtained in the past by a Pechmann condensation to give either a 4-position methyl group or a 3-position alkyl substitution on the coumarin [H.V. Pechmann and C. Duisberg, *Chem. Ber.* **16**, 2119 1883)]: 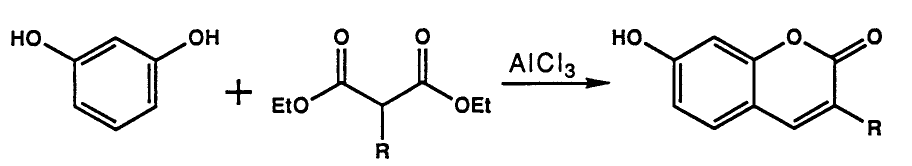
Where R represents the activatable tether.

The 4-methyl product does not provide an activatable tether group. The effect of 3-position substitution on electronic structure in coumarins is shown in the ¹³C NMR spectra compiled by Parmar and Boll [*Mag. Res. Chem.,* **26**, 430-433 (1988)]. If **R** of the product above is **H**, the ¹³C NMR chemical shift of C-3 is 110 ppm; while if **R** is **CH₂COOEt**, C-3 resonates at 115 ppm. This means that the relative electron density on C-3 has decreased upon alkyl substitution, disturbing the electronic structure of the coumarin nucleus. Thus, the Pechmann condensation was not useful since it did not produce 4-substituted materials, exclusive of having substitution at the 3-position, and since a compound having no substituent at the 3-position was desired because of the need for substantially similar electronic properties as 4-MU.

Of interest as background art is Biochemistry 24(3), 573-81 (1985) which discloses 4-heptadecyl-7-hydroxycoumarin as a fluorophore that is useful as a probe to study the properties of phospholipid bilayers at the lipid-water interface. Steady-state fluorescence anisotropy, differential polarized phase fluorometry, and emission lifetime of the above compound were measured in isotropic viscous medium, in lipid vesicles, and in the membrane of vesicular stomatitus virus.

Also of interest as background to the present invention is US Patent 4,618,622 which discloses substituted hydroxycoumarins as intermediates in the synthesis of the corresponding pharmaceutically active sulfonates.

### Summary of the Invention

In one aspect, the invention relates to a compound of the formula: 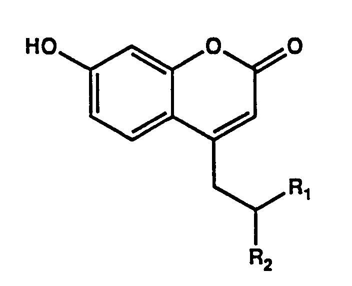
wherein R₁ is selected from the group consisting of H, -CH₃, -G-OZ, -G-SZ, -G-NHY, -SZ, -NHY, substituted phenyl, and substituted or unsubstituted alkyl of the formula -G-CH₃, where G represents an alkylene chain having from 1 to 25 carbon atoms, and Z represents a protecting group for O or S, and Y represents a protecting group for N; and
wherein R₂ is selected from the group consisting of -J-OH, -J-SH, -J-NH₂, -J-OTs, -J-X, -SH, -NH₂, and -COOR', where J represents an alkylene chain having from 1 to 10 carbon atoms, X represents a halogen, and R' represents hydrogen or an alkyl chain having from 1 to 10 carbon atoms, provided that when R₁ is -CH₃ then R₂ is -COOH or -COOCH₂CH₃.

In another aspect, the invention relates to a process of synthesis for the compounds described above.

The steps of the synthesis comprise:
a) reacting 4-bromomethyl-7-methoxycoumarin with a monoalkylated (R₁) malonic ester under conditions sufficient to achieve condensation of the ester to give the monoalkylated (2-bis(carbalkoxy)-1-ethyl) derivative;
b) removing one of the carbalkoxy groups from the product of step a);
c) demethylation of the product of step b) to give the 7-hydroxycoumarin compound; and
d) chemically modifying the remaining ester to yield a desired R₂
Preferably, step b) is performed according to the process of Krapcho in the presence of NaCl and DMSO at high temperatures. It is also preferred that step c) is performed by reacting ethanethiol (EtSH) with the product of step b) at 0°C in the presence of AlCl₃ and dichloromethane.

Finally, the invention also comprises a method of using the compounds described above. The 7-hydroxy-4-methyl coumarins are known to fluoresce. By conjugating the compounds to a biological macromolecule, the presence or absence of the macromolecule can be quantified. For example, a method of using compounds according to the invention comprises:
a) coupling the compound to a biological macromolecule to be used in a reaction of interest; and
b) determining the amount of macromolecule by measuring the fluorescence of the compound.

Preferably, the compounds according to the invention are conjugated to a member of a specific binding pair, such as an antibody or antigen for determination in an immunoassay. They may also be conjugated to oligonucleotides and used in PCR or other hybridization assays.

### Detailed Description of the Invention

The invention comprises compositions of matter, processes of synthesis and methods of use for the compounds.

### Compounds:

In one aspect, the invention relates to compounds having the general formula: 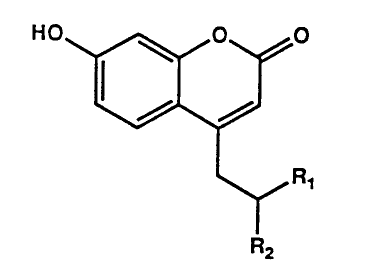
wherein R₁ is selected from the group consisting of H, -CH₃, -G-OZ, -G-SZ, -G-NHY, -SZ, -NHY, substituted phenyl, and substituted or unsubstituted alkyl of the formula -G-CH₃, where G represents an alkylene chain having from 1 to 25 carbon atoms, and Z represents a protecting group for O or S, and Y represents a protecting group for N; and
wherein R₂ is selected from the group consisting of -J-OH, -J-SH, -J-NH₂_{,} -J-OTs, -J-X, -SH, -NH₂, and -COOR', where J represents an alkylene chain having from 1 to 10 carbon atoms, X represents a halogen, and R' represents hydrogen or an alkyl chain having from 1 to 10 carbon atoms, provided that when R₁ is -CH₃ then R₂ is -COOH or -COOCH₂CH₃.

Arbitrarily, R₁ derives from the central, monoalkylated R₁ of the malonic ester; while R₂ is converted from the COOEt end chain of the ester. R₁ can be selected from any of the groups listed above, although H is preferred. Other R₁ groups may require protecting groups to enable them to withstand the ensuing reactions. As used herein, "protecting group" refers to any group that can be attached to a functional moiety permitting it to withstand future reaction conditions without destroying the function; and which later can be removed or substituted to give back the functional group. For example, if alcohol or thiol groups are used, a protective group Z is used. In the case of alcohols, Z may be t-butyldimethylsilyl or tetrahydropyran; while for thiols, a preferred Z is triphenylmethyl. Protective group Y is similarly required for amino substituents. In this case, acetyl is a preferred protecting group. It is to be understood, of course, that other protecting groups are known in the art, and are obvious extensions falling within the scope of the invention.

R₂ can be a greater number of groups since it is converted from the COOEt ester after the other reactions are completed. Conventional organic chemistry methods can place almost any group in the R₂ position, although there is little practical reason why some groups would be made. The preferred group will be dictated by the linking moiety present on the biological macromolecule of interest (see below). For example, if the macromolecule contains a primary amine, it is preferred that R₂ be (or be converted to) a N-hydroxysuccinimide ester. Other preferred R₂ groups are given in Table 1 below. A tosyl group, Ts, may also be created at R₂ and is useful as an intermediate to create other R₂ groups as shown in the Examples.

As used herein, "alkylene" refers to any straight or branched chain spacer group containing less than 50 carbon atoms, including but not limited to, -CH₂-, -CH(CH₃)-, -CH(C₂H₅)-, -CH(CH₃)CH₂-, -(CH₂)₃-, and the like. The length of the alkylene chain is preferably short to enhance solubility, to avoid steric problems, and to be readily available commercially. Ideally, the alkylene chain G should be from 1 to about 10 carbon atoms long, while the alkylene chain J should be from 1 to about 5 carbon atoms long. In either case, the alkylene chain may be substituted.

"Aryl" refers to substituents having ring structures. For solubility reasons, phenyl or substituted phenyl is preferred over larger aryl groups.

Both aryl and alkylene substituents at the R₁ position may be substituted. As used herein, "substituted" refers to the presence of moieties covalently bonded to the aryl or alkylene groups, including, but not limited to, halide (especially Br and Cl), nitro, lower alkoxy (having from 1-6 carbon atoms, especially methoxy and ethoxy), lower alkyl (having from 1-6 carbon atoms, especially methyl and ethyl), hydroxy, and amino (protecting group may be required). Subject to the limits of organic chemistry, the substituting groups may be placed anywhere, and in any number, on the alkylene or phenyl substituent.

It should be recalled that the object of the invention was to put a tether group in the 4 position of the coumarin nucleus. Therefore, if R₁ is H, it is pointless to convert R₂ to H or alkyl since there would then be no functional group to serve as a tether. It is important to the invention that at least one of R₁ and R₂ provide a functional group that is, or can be activated to be, reactive with a biological macromolecule or a linker as described below.

The compounds of the present invention find utility as fluorophores. Specifically, the side chains in the 4 position enable the compounds to be covaiently coupled to other molecules through conventional chemistries, without affecting the electron configurations that are responsible for their fluorometric properties. For example, the 7-hydroxy-2-oxo-2H-1-benzopyran-4-propionic acid was synthesized for the purpose of covalently labeling biological macromolecules such as oligonucleotide primers.

The labeled macromolecules can then be detected by any fluorometric procedure, such as on an IMₓ® instrument, without recourse to enzymatic signal amplification.

The compounds can also be used as dye markers at the 5' end of an oligonucleotide for sequencing purposes.

### Process of Synthesis:

The compounds of the present invention can be chemically synthesized in 3 or more steps starting from 4-bromomethyl-7-methoxycoumarin 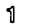 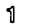 (Aldrich Chemical Co., Milwaukee, WI). The compounds were prepared as shown below. Generally speaking, malonic ester displacement of the bromide in 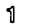 , in the presence of NaH, afforded monoalkylated 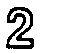 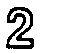 according to the following reaction: 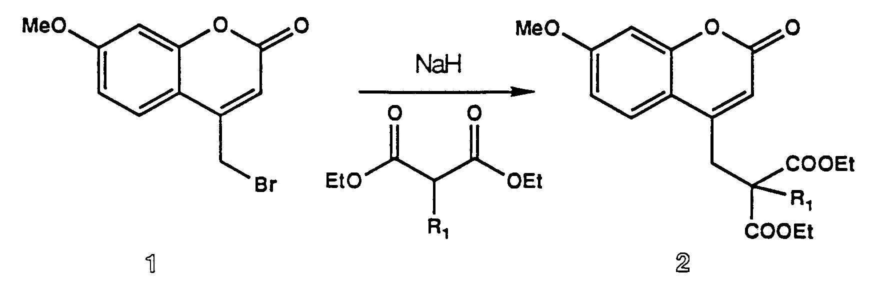
Krapcho decarbalkoxylation [Krapcho, A. P.; Weimaster, J. F.; Eldridge, J. M.; Jahngen, Jr., E. G. E.; Lovey, A. J.; Stephens, W. D. *J. Org. Chem*. (1978) **43**: 138-147] removed one of the two ester groups to give 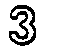 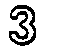 in good yield. The reaction is best carried out at high temperatures using NaCl as follows: 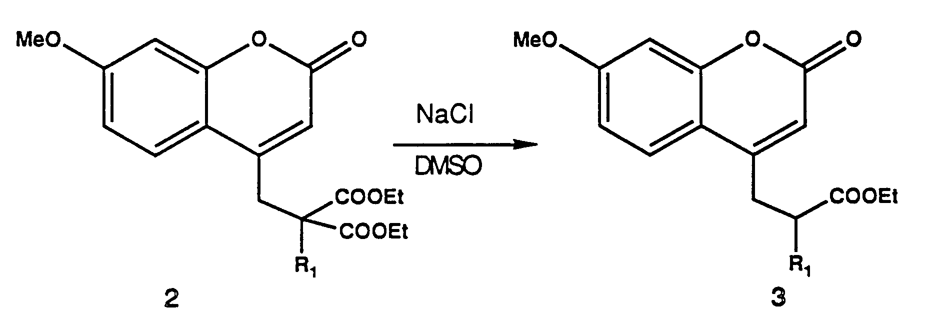
Demethylation of 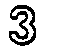 to give the 7-hydroxycoumarin was accomplished under the conditions of Fujita as described in [Node, et al *J. Org. Chem*. (1980) **45**: 4275-4277]. Briefly, the conditions involve a strong Lewis acid, AlCl3, and ethane thiol (EtSH) as a weak Lewis base, a source of protons, and a soft nucleophile. After several other methods {including BBr₃, [McOmie, et al. *Tetrahedron* (1968) **24**: 2289-2292]; Me₃Sil,[Ho, et al, *Angew. Chem*. (1976) **88**: 847]; and NaSEt [Feutrill, et al, *Tetrahedron Letters* (1970) **161**: 327-328]} failed, the conditions of Fujita finally effected demethylation to give 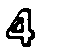 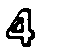 , albeit in only low yields. 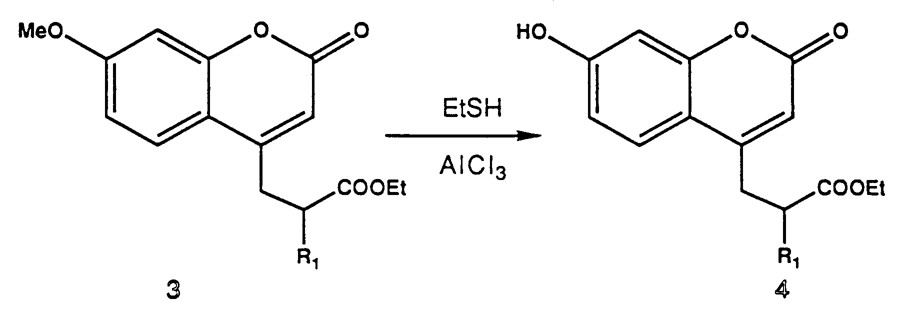
The ester is among the claimed R₂ groups. To arrive at the other R₂ groups, conventional organic chemistry can be used, directly from the ester or from other intermediate groups. For example, saponification of 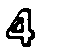 gave the acid 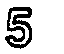 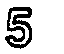 as shown below. 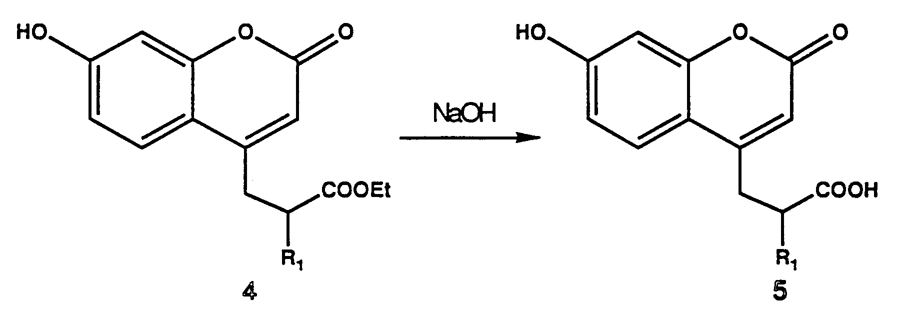
From either the ester or the acid, the remaining R₂ groups can be obtained through the following reactions. Where R₂ is to be an alcohol, LiBH4 reduction gives the desired product. The alcohol hydroxyl can then be converted to either the thiol or the amino through the intermediate hydroxy tosylate. The ester may also be reduced to the aldehyde using diisobutylaluminum hydride (DIBAL, Aldrich Chemical Co.). Longer alkylene chains can be synthesized from the aldehyde using an appropriate Wittig or Wadsworth/Emmons reagent.

### Methods of Use:

Finally, the invention also comprises a method of using the compounds described above. The 7-hydroxy-4-methyl coumarins are known to fluoresce. By conjugating the compounds to a biological macromolecule, the presence or absence of the macromolecule can be quantified. For example, compounds according to the invention may be conjugated to antibodies for determination in an immunoassay. They may also be conjugated to oligonucleotides and used in PCR or other nucleic acid hybridization assays.

The conjugation is generally carried out by activating the fluorophore with a reactive group. In the case of a carboxyl R₂ to be reacted with a primary amine on a target macromolecule, the preferred activator is N-hydroxysuccinimide ester. Other activating groups are known in the art for use with the various R₂ groups and various target macromolecule linking moieties. Table 1 below is a nonexhaustive listing of some exemplary target moieties, likely R₂ groups and useful activators. In some cases, the conjugation is best performed using a linker or spacer molecule. The linker may be heterobifunctional or homobifunctional depending on the circumstances. The correct linker can also be determined by one of ordinary skill in the art.

**Table 1**

| **Target Moiety** *(on biomolecule)* | **Linker** | **Preferred R₂ Group** | **Activator** |
|---|---|---|---|
| amine | none | carboxyl | NHS-ester |
| amine | maleimide | thiol | none (stable linkage) |
| amine | thiol | thiol | none (easily reversible linkage) |
| carboxyl | none | amine | (carbodiimide) |
| *vicinal* diol | (oxidation) | amine | (cyanoborohydride reduction *after* linkage) |
| thiol | none | amine | maleimide (stable linkage) |
| thiol | none | thiol | none (easily reversible linkage) |
| hydroxyl | (convert to tosyl) | amine | none |
| hydroxyl | (convert to phosphate ester) | hydroxyl | none |

The invention will now be further described by way of Examples.

### Examples

### Example 1: Synthesis of 4-(2-carboxy-1-ethyl)-7-hydroxy-2-oxo-2H-1-Benzopyran

Throughout this example, R₁ represents Hydrogen.

### A. Materials and Methods.

Chemical reagents were purchased from Aldrich. Proton NMR spectra were obtained at 300 MHz on a General Electric QE-300 spectrometer, referenced to TMS internal standard in ppm (δ). Coupling constants are given in hertz. Mass Spectra were obtained using direct chemical ionization on a Kratos MS 50 instrument. Aminomodifier II was purchased from Clontech Laboratories (Palo Alto, CA). TLC analyses were done using 250 µm Analtech silica gel plates. Flash column chromatographies were run with EM Kieselgel60 (70-230 mesh). Spectral and elemental analyses were performed by the Analytical Research Department, Abbott Laboratories.

*B. Synthesis of 4-(2-bis(carbethoxy)-1-ethyl)-7-methoxy-2-oxo*-*2H-1-Benzopyran* ( 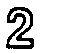 )

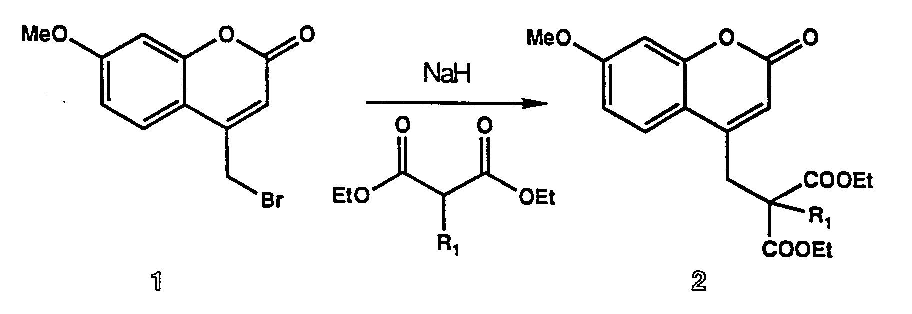

To a suspension of 240 mg of 60% NaH mineral oil dispersion (6 mmol) in 10 mL of DMF was added 961 µL (6mmol) of diethyl malonate. After the foaming subsided and the suspension cleared to a solution, 1346 mg (5mmol) of 4-bromomethyl-7-methoxycoumarin was added all at once. After stirring for 4 h at room temperature, the DMF was stripped off, and the residue partitioned between 0.01 M HCl/hexane. The organic phase was concentrated and vacuum dried, then as much as possible was taken up into 4 mL of 50/50 EtOAC/hexane. Flash chromatography gave 670 mg of 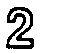 as a white solid, 49%.

Analysis gave:
1H NMR (CDCl₃) δ 7.56 (d, 1 H, J=8.8), 6.88 (dd, 1 H, J=2.6, 8.8), 6.84 (br s, 1H), 4.23 (q, 2 H, J=7.2), 4.22 (q, 2 H, J=7.2), 3.88 (s, 3 H), 3.74 (t, 1 H, J=7.4), 3.36 (dd, 2 H, J=1.1, 7.4), 1.27 (t, 6 H, J=7.4)
MS m/z 349 (100, M+H)
IR (film, cm-1) 1715 (vs), 1614 (vs)
Anal. (C₁₈H₂₀O₇) C, H.

*C. Synthesis of 4-(2-carbethoxy-1-ethyl)-7-methoxy-2-oxo-2H-1-Benzopyran* ( 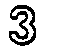 )

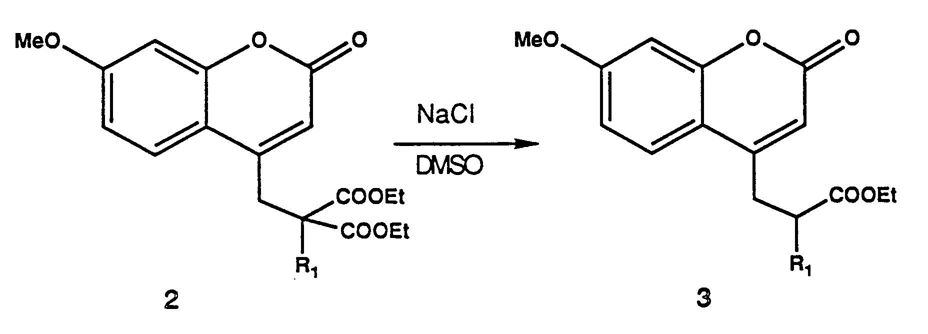

To a solution of 44.8 mg (0.13 mmol) of 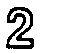 in 6 mL of DMSO was added 15 mg of NaCl, followed by 4.6 mL of water. The reaction was stirred in an oil bath at 180°C for 2.5 h, and was cooled to room temperature. After addition of 45 mL of water to the reaction mixture, the resultant emulsion was extracted with 2x40 mL EtOAc. The organic phase was concentrated by rotary evaporation, and was vacuum dried. After uptake into 3 mL of 25% EtOAC in hexane, flash chromatography using the same solvent system gave 31.4 mg of 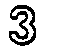 , 88%.

Analysis gave:
1H NMR (CDCl₃) δ 7.55 (d, 1H, J=8.8), 6.88 (dd, 1H, J=2.6, 8.8), 6.83 (d, 1H, J=2.6), 6.13 (t, 1H, J=1.1), 4.19 (q, 2H, J=7), 3.88 (s, 3H), 3.06 (t, 2H, J=8), 2.71 (t, 2H, J=8), 1.28 (t, 3H, J=7)
MS 277 (100, M+H)
IR (film, cm-1) 1730(vs), 1612(vs)
Anal. (C₁₅H₁₆O₅) C, H.

*D. Synthesis of 4-(2-carbethoxy-1-ethyl)-7-hydroxy-2-oxo-2H-1-Benzopyran* ( 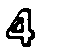 )

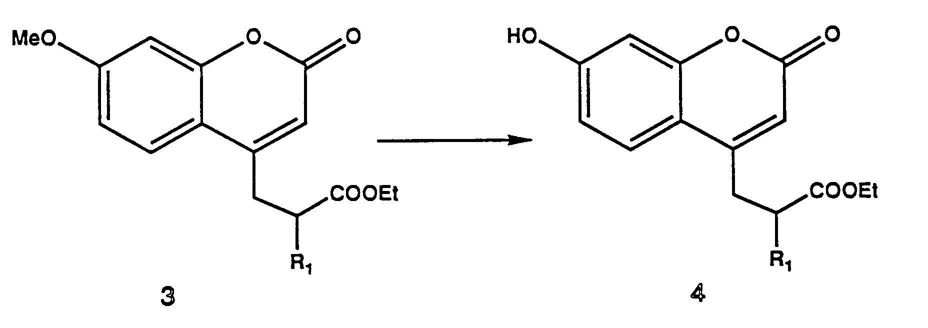

To a suspension of 726 mg (5.4 mmol) of AlCl₃ in 10 mL of dichloromethane at 0°C was added 4 mL of EtSH. The suspension became a clear solution within seconds. Then, 298 mg (1.08 mmol) of 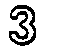 in 4 mL of dichloromethane was added, turning the yellow solution red in color. The ice bath was removed, and the reaction stirred to room temperature for 3 h. The solvents were removed in vacuo, and the residue thoroughly vacuum dried. The residue was extracted into EtOAC as much as possible, then the extract was flash chromatographed using 30/70 EtOAc/hexane. The long- and shortwave UV active band gave 76.7 mg (27%) of 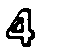 .

Analysis gave:
1H NMR (CDCl₃) δ 7.5 (d, 1H, J=8.5), 6.9 (d, 1H, J=2.6), 6.85 (dd, 1H, J=8.5, 2.6), 6.1 (br s, 1H, 4.18 (q, 2H, J=7.4), 3.08 (br t, 2H, J=7), 2.71 (br t, 2H, J=7), 1.28 (t, 3H, J=7.4)
MS 263 (M+H)
IR (film, cm-1) 3280 (s, br), 1730 (vs), 1693 (vs), 1608 (vs), 1565 (s)
Anal. (C₁₄H₁₄O₅) C,H.

*E. Synthesis of 4-(2-carboxy-1-ethyl)*-*7*-*hydroxy-2-oxo-2H-1-Benzopyran* ( 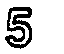 )

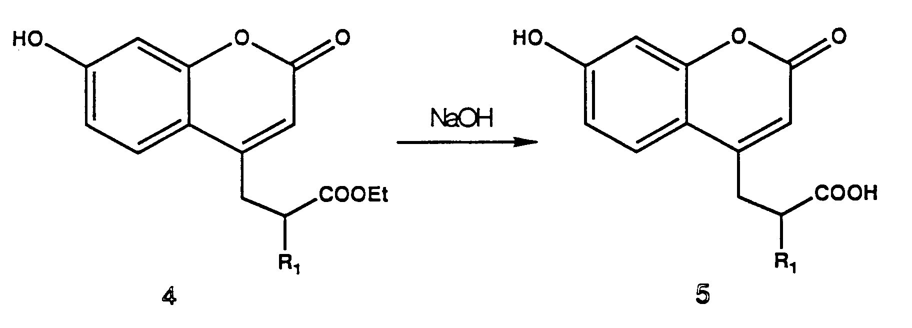

A 36.7 mg sample of ester 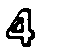 was suspended into 10 mL of water, and 25 mL of 50% aqueous NaOH was added. The resultant solution was stirred at room temperature for 4 h. TLC analysis (30/70 EtOAc/hexane) after this time showed that no starting material remained. The mixture was acidified using 1 mL of 1 M HCl. A precipitate formed upon acidification, and the white solid was left to deposit for 1 h. After the solid was filtered off and thoroughly washed with 1 M HCl, it was vacuum dried to give 14.1 mg (43%) of analytically pure 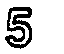 .

Analysis gave:
1H NMR (NaOD/D₂O) δ 7.5 (d, 1H, J=8.8), 6.74 (dd, 1H, J=2.2, 8.8), 6.53 (d, 1H, J=2.2), 5.97 (br s, 1H), 2.91 (t, 2H, J=7.4), 2.5 (t, 2H, J=7.4) MS (FAB, H2O) 235 (M+H)
IR (KBr, cm-1) 3440 (s, br), 1710 (vs), 1611 (vs), 1568 (vs), 1400 (s)
Anal. (C₁₂H₁₀O₅) C, H.

*F. Synthesis of 4*-*(2*-*carboxy*-*1*-*ethyl)*-*7-hydroxy-2-oxo-2H-1-Benzopyran, N-hydroxy succinimide ester* ( 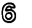 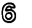 ).

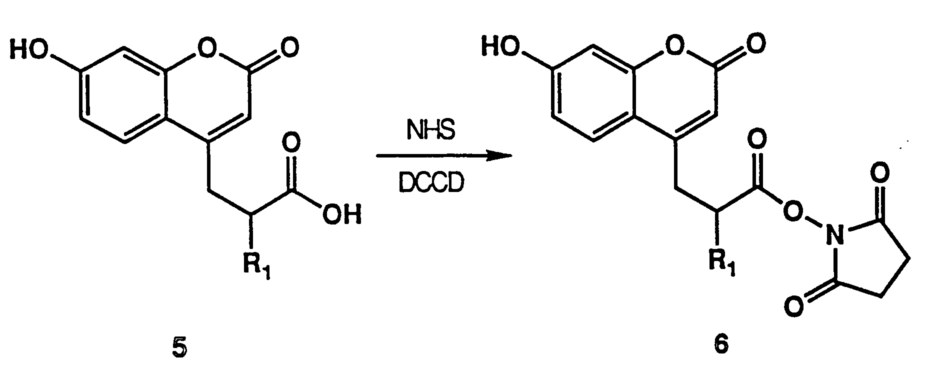

To a suspension of 6.4 mg (27.3 mmol) of 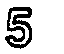 in 6 mL of MeCN was added 4.7 mg (41 mmol) of N-hydroxysuccinimide, 8.4 mg (41mmol) of dicyclohexylcarbodiimide (DCCD) and 2 mg of 4,4-dimethylaminopyridine. The reaction was stirred at room temperature for 24 h, and the solvent was removed in vacuo. The residue was taken up into 750 µL of DMF, and coupled directly with oligonucleotide.

### Example 2: Synthesis of 4-(2-carboxy-2-R₁-1-ethyl)-7-hydroxy-2-oxo-2H-1-Benzopyran

Example 1 is repeated with R₁ groups according to Table 2.

**Table 2**

| R₁ group |
|---|
| -CH₃ |
| -CH₂CH₃ |
| -CH₂CH₂₋O-t-butyldimethylsilyl |
| -CH₂CH₂₋NH-acetyl |
| -NH-acetyl |

### Example 3: Synthesis of Alcoholic R₂ Group

Compound ( 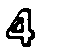 ) from Example 1 is modified to contain an alcoholic R₂ group (-CH₂OH) by reducing the ester with LiBH₄ under conditions cited by Brown in: H.C. Brown, *Hydroboration*, p. 245, Benjamin, NY, NY (1962).

### Example 4: Synthesis of Thiol R₂ Group

The compound from Example 3 is modified to contain a thiol R₂ group (-CH₂SH) by conversion using the tosylate under conditions of Price and Stacy in: *Organic Synthesis Collective vol.* **3**, p.86 (1955).

### Example 5: Synthesis of Amine R₂ Group

The compound from Example 3 is modified to contain an amino R₂ group (-CH₂NH₂) by conversion using the tosylate under conditions of a Gabriel synthesis of primary amines, wherein the tosylate is displaced by sodium phthalimide. The phthalimide is then removed with hydrazine to give the primary amine.

The above examples serve to illustrate the invention and should not be construed as limiting the scope of the invention. Rather, the scope of the invention is limited only by the appended claims.

## Claims (Claims for the following Contracting State(s): DE, FR, IT)

1. A compound of the formula: 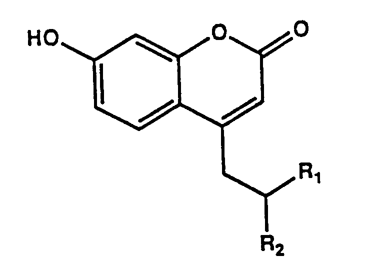 wherein R₁ is selected from H, -CH₃, -G-OZ, -G-SZ, -G-NHY, -SZ, -NHY, substituted phenyl, and substituted or unsubstituted alkyl of the formula -G-CH₃, where G represents an alkylene chain having from 1 to 25 carbon atoms, and Z represents a protecting group for O or S, and Y represents a protecting group for N; and
wherein R₂ is selected from -J-OH, -J-SH, -J-NH₂, -J-OTs, -J-X, -SH, -NH₂, and -COOR', where J represents an alkylene chain having from 1 to 10 carbon atoms, X represents a halogen, and R' represents hydrogen or an alkyl chain having from 1 to 10 carbon atoms, provided that when R₁ is -CH₃ then R₂ is -COOH or -COOCH₂CH₃.

2. A compound according to claim 1, wherein Z is selected from t-butyldimethylsilyl, tetrahydropyran, and triphenylmethyl.

3. A compound according to claim 1 or 2, wherein Y is acetyl.

4. A compound according to any of claims 1 to 3, wherein R₁ is alkyl and G is an alkylene chain having from 1 to 10 carbon atoms.

5. A compound according to any of claims 1 to 4, wherein J is an alkylene chain having from 1 to 5 carbon atoms.

6. A compound according to claim 1, wherein R₂ is -COOH.

7. A compound according to claim 1 or 4, wherein R₁ is selected from methyl, ethyl and isopropyl.

8. A compound according to any of claims 1 to 4, wherein R₂ is -COOR', where R' represents hydrogen or an alkyl chain having from 1 to 10 carbon atoms.

9. A process for making a compound of the formula: 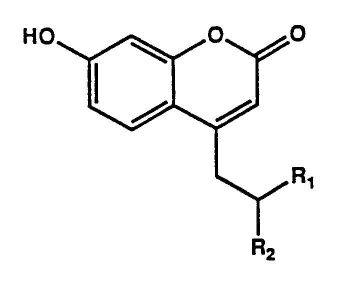 wherein R₁ is selected from H, -CH₃, -G-OZ, -G-SZ, -G-NHY, -SZ, -NHY, substituted phenyl, and substituted or unsubstituted alkyl of the general formula -G-CH₃, where G represents an alkylene chain having from 1 to 25 carbon atoms, and Z represents a protecting group for O or S, and Y represents a protecting group for N; and
wherein R₂ is selected from -J-OH, -J-SH, -J-NH₂, -J-OTs, -J-X, -SH, -NH₂ and -COOR', where J represents an alkylene chain having from 1 to 10 carbon atoms, X represents a halogen, and R' represents hydrogen or an alkyl chain having from 1 to 10 carbon atoms, provided that when R₁ is -CH₃ then R₂ is -COOH or -COOCH₂CH₃,
said process comprising the steps of:
a) reacting 4-bromomethyl-7-methoxycoumarin with a malonic ester under conditions sufficient to achieve condensation of the ester to give the (2-bis(carbalkoxy)-2-R₁-1-ethyl) coumarin derivative;
b) removing one of the carbalkoxy groups from the product of step a);
c) demethylation of the product of step b) to give the 7-hydroxycoumarin compound; and
d) chemically modifying the remaining carbalkoxy ester to yield a desired R₂.

10. A process according to claim 9, wherein step d) comprises modifying the remaining ester to convert it to a member selected from -J-OH, -J-SH, -J-NH₂, -J-OTs, -J-X, -SH, NH₂ and -COOR', where J represents an alkylene chain having from 1 to 5 carbon atoms, X represents a halogen, and R' represents hydrogen or an alkyl chain having from 1 to 10 carbon atoms.

11. A process according to claim 9 or 10, wherein step d) comprises saponification of the remaining ester to a carboxylic acid.

12. A process according to claim 9 or 10, wherein step d) comprises modifying the remaining ester to an amino group.

13. A process according to claim 9 or 10, wherein step d) comprises modifying the remaining ester to a hydroxyl group.

14. A process according to any of claims 9 to 13, wherein step b) is performed in the presence of NaCl and DMSO at high temperatures.

15. A process according to any of claims 9 to 14, wherein step c) is performed in the presence of a strong Lewis acid and a weak nucleophilic Lewis base.

16. A process of labeling a biological macromolecule comprising covalently conjugating a compound according to any of claims 1 to 8 to a biological macromolecule using R₂ and optionally an activator to link said product to said macromolecule.

17. A method of using compounds according to any of claims 1 to 8 comprising the steps of:
a) coupling the compound to a biological macromolecule to be used in a reaction of interest; and
b) determining the amount of such macromolecule by measuring the fluorescence of the compound.

18. A conjugate comprising a compound according to any of claims 1 to 8, wherein R₂ is coupled with a biological macromolecule.

19. The invention according to any of claims 16 to 18, wherein said biological macromolecule is a member of a specific binding pair.

20. The invention according to claim 19, wherein said member of a specific binding pair is selected from an antibody, an antigen, and a hapten.

21. The invention according to claim 19, wherein said member of a specific binding pair is an oligonucleotide.

## Claims (Claims for the following Contracting State(s): ES)

1. A process for making a compound of the formula: 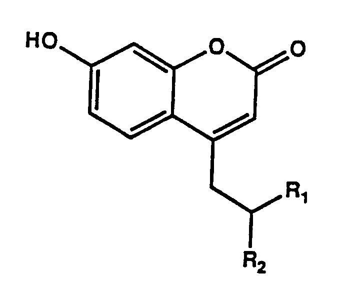 wherein R₁ is selected from H, -CH₃, -G-OZ, -G-SZ, -G-NHY, -SZ, -NHY, substituted phenyl, and substituted or unsubstituted alkyl of the general formula -G-CH₃, where G represents an alkylene chain having from 1 to 25 carbon atoms, and Z represents a protecting group for O or S, and Y represents a protecting group for N; and
wherein R₂ is selected from -J-OH, -J-SH, -J-NH₂, -J-OTs, -J-X, -SH, -NH₂ and -COOR', where J represents an alkylene chain having from 1 to 10 carbon atoms, X represents a halogen, and R' represents hydrogen or an alkyl chain having from 1 to 10 carbon atoms, provided that when R₁ is -CH₃ then R₂ is -COOH or -COOCH₂CH₃,
said process comprising the steps of:
a) reacting 4-bromomethyl-7-methoxycoumarin with a malonic ester under conditions sufficient to achieve condensation of the ester to give the (2-bis(carbalkoxy)-2-R₁-1-ethyl) coumarin derivative;
b) removing one of the carbalkoxy groups from the product of step a);
c) demethylation of the product of step b) to give the 7-hydroxycoumarin compound; and
d) chemically modifying the remaining carbalkoxy ester to yield a desired R₂.

2. A process according to claim 1, wherein step d) comprises modifying the remaining ester to convert it to a member selected from -J-OH, -J-SH, -J-NH₂, -J-OTs, -J-X, -SH, NH₂ and -COOR', where J represents an alkylene chain having from 1 to 5 carbon atoms, X represents a halogen, and R' represents hydrogen or an alkyl chain having from 1 to 10 carbon atoms.

3. A process according to claim 1 or 2, wherein step d) comprises saponification of the remaining ester to a carboxylic acid.

4. A process according to claim 1 or 2, wherein step d) comprises modifying the remaining ester to an amino group.

5. A process according to claim 1 or 2, wherein step d) comprises modifying the remaining ester to a hydroxyl group.

6. A process according to any of claims 1 to 5, wherein step b) is performed in the presence of NaCl and DMSO at high temperatures.

7. A process according to any of claims 1 to 6, wherein step c) is performed in the presence of a strong Lewis acid and a weak nucleophilic Lewis base.

8. A process of labeling a biological macromolecule comprising covalently conjugating the product of step d) according to any of claims 1 to 7 to a biological macromolecule using R₂ and optionally an activator to link said product to said macromolecule.

9. A method of using a compound of the formula 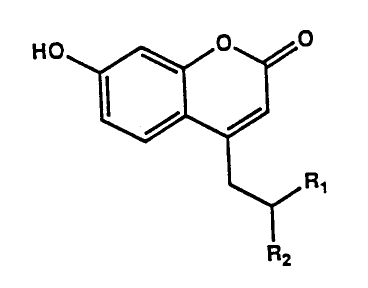 wherein R₁ is selected from H, -CH₃, -G-OZ, -G-SZ, -G-NHY, -SZ, -NHY, substituted phenyl, and substituted or unsubstituted alkyl of the formula -G-CH₃, where G represents an alkylene chain having from 1 to 25 carbon atoms, and Z represents a protecting group for O or S, and Y represents a protecting group for N; and
wherein R₂ is selected from -J-OH, -J-SH, -J-NH₂, -J-OTs, -J-X, -SH, -NH₂, and -COOR', where J represents an alkylene chain having from 1 to 10 carbon atoms, X represents a halogen, and R' represents hydrogen or an alkyl chain having from 1 to 10 carbon atoms, provided that when R₁ is -CH₃ then R₂ is -COOH or -COOCH₂CH₃,
said method comprising the steps of:
a) coupling the compound to a biological macromolecule to be used in a reaction of interest; and
b) determining the amount of such macromolecule by measuring the fluorescence of the compound.

10. A method according to claim 9, wherein Z is selected from t-butyldimethylsilyl, tetrahydropyran, and triphenylmethyl.

11. A method according to claim 9 or 10, wherein Y is acetyl.

12. A method according to any of claims 9 to 11, wherein R₁ is alkyl and G is an alkylene chain having from 1 to 10 carbon atoms.

13. A method according to any of claims 9 to 12, wherein J is an alkylene chain having from 1 to 5 carbon atoms.

14. A method according to claim 9, wherein R₂ is -COOH.

15. A method according to claim 9 or 12, wherein R₁ is selected from methyl, ethyl and isopropyl.

16. A method according to any of claims 9 to 12, wherein R₂ is -COOR', where R' represents hydrogen or an alkyl chain having from 1 to 10 carbon atoms.

17. The invention according to claims 8 or 9, wherein said biological macromolecule is a member of a specific binding pair.

18. The invention according to claim 17, wherein said member of a specific binding pair is selected from an antibody, an antigen, and a hapten.

19. The invention according to claim 17, wherein said member of a specific binding pair is an oligonucleotide.

## Patentansprüche (Patentansprüche für folgende(n) Vertragsstaat(en): DE, FR, IT)

1. Verbindung nach folgender Formel: 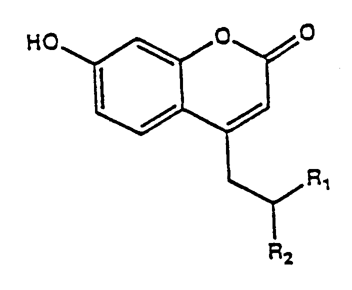 worin R₁ aus H, -CH₃, -G-OZ, -G-SZ, G-NHY, -SZ, -NHY, substituiertem Phenyl und aus substituiertem oder unsubstituiertem Alkyl der Formel -G-CH₃ gewählt ist, wobei G eine Alkylenkette mit 1 bis 25 Kohlenstoffatomen darstellt, und wobei Z eine Schutzgruppe für O oder S darstellt, und wobei Y eine Schutzgruppe für N darstellt; und
worin R₂ aus -J-OH, -J-SH, -J-NH₂, -J-OTs, -J-X, -SH, -NH₂ und -COOR' gewählt ist, worin J eine Alkylenkette mit 1 bis 10 Kohlenstoffatomen darstellt, worin X ein Halogen darstellt, und worin R' Wasserstoff oder eine Alkylkette mit 1 bis 10 Kohlenstoffatomen darstellt, vorausgesetzt, daß wenn R₁ gleich - CH₃ ist, dann R₂ gleich -COOH oder -COOCH₂CH₃ ist.

2. Verbindung nach Anspruch 1, worin Z aus t-Butyldimethylsilyl, Tetrahydropyran und Triphenylmethyl gewählt ist.

3. Verbindung nach einem oder mehreren der Ansprüche 1 oder 2, worin Y gleich Acetyl ist.

4. Verbindung nach einem oder mehreren der Ansprüche 1 bis 3, worin R₁ gleich Alkyl ist und G eine Alkylenkette mit 1 bis 10 Kohlenstoffatomen ist.

5. Verbindung nach einem oder mehreren der Ansprüche 1 bis 4, worin J eine Alkylenkette mit 1 bis 5 Kohlenstoffatomen ist.

6. Verbindung nach Anspruch 1, worin R₂ gleich -COOH ist.

7. Verbindung nach Anspruch 1 oder 4, worin R₁ aus Methyl, Ethyl und Isopropyl ausgewählt ist.

8. Verbindung nach einem oder mehreren der Ansprüche 1 bis 4, worin R₂ gleich -COOR' ist, wobei R' Wasserstoff oder eine Alkylkette mit 1 bis 10 Kohlenstoffatomen darstellt.

9. Verfahren zur Herstellung einer Verbindung nach folgender Formel: 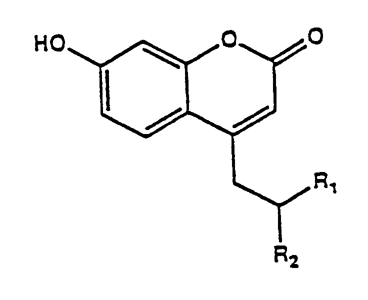 worin R₁ aus H, -CH₃, -G-OZ, -G-SZ, G-NHY, -SZ, -NHY, substituiertem Phenyl und aus substituiertem oder unsubstituiertem Alkyl der allgemeinen Formel -G-CH₃ gewählt ist, wobei G eine Alkylenkette mit 1 bis 25 Kohlenstoffatomen darstellt, und wobei Z eine Schutzgruppe für O oder S darstellt, und wobei Y eine Schutzgruppe für N darstellt; und
worin R₂ aus -J-OH, -J-SH, -J-NH₂, -J-OTs, -J-X, -SH, -NH₂ und -COOR' gewählt ist, worin J eine Alkylenkette mit 1 bis 10 Kohlenstoffatomen darstellt, worin X ein Halogen darstellt, und worin R' Wasserstoff oder eine Alkylkette mit 1 bis 10 Kohlenstoffatomen darstellt, vorausgesetzt, daß wenn R₁ gleich - CH₃ ist, dann R₂ gleich -COOH oder -COOCH₂CH₃ ist,
wobei das Verfahren folgende Schritte umfaßt:
a) Umsetzen von 4-Brommethyl-7-methoxycrnmarin mit einem Malonester unter Bedingungen, die zur Durchführung der Esterkondensation ausreichend sind unter Erzeugung des (2-Bis(carbalkoxy)-2-R₁-1-ethyl)cumarinderivats;
b) Entfernen einer der Carbalkoxygruppen aus dem Produkt nach Schritt a);
c) Demethylieren des Produktes nach Schritt b) unter Erzeugung der 7-Hydroxycumarinverbindung; und
d) chemisches Abwandeln des verbleibenden Carbalkoxyesters unter Erzeugung eines gewünschten R₂.

10. Verfahren nach Anspruch 9, worin Schritt d) das Abwandeln des verbleibenden Esters zu seiner Überführung in ein Glied umfaßt, das aus -J-OH, -J-SH, -J-NH₂, -J-OTs, -J-X, -SH, -NH₂ und -COOR' gewählt ist, worin J eine Alkylenkette mit 1 bis 5 Kohlenstoffatomen darstellt, worin X ein Halogen darstellt, und worin R' Wasserstoff oder eine Alkylkette mit 1 bis 10 Kohlenstoffatomen darstellt.

11. Verfahren nach Anspruch 9 oder 10, worin Schritt d) die Verseifung des verbleibenden Esters zu einer Carbonsäure umfaßt.

12. Verfahren nach Anspruch 9 oder 10, worin Schritt d) das Abwandeln des verbleibenden Esters zu einer Aminogruppe umfaßt.

13. Verfahren nach Anspruch 9 oder 10, worin Schritt d) das Abwandeln des verbleibenden Esters zu einer Hydroxylgruppe umfaßt.

14. Verfahren nach einem oder mehreren der Ansprüche 9 bis 13, worin Schritt b) in Gegenwart von NaCl und DMSO bei hohen Temperaturen durchgeführt wird.

15. Verfahren nach einem oder mehreren der Ansprüche 9 bis 14, worin Schritt c) in Gegenwart einer starken Lewis-Säure und einer schwachen nukleophilen Lewis-Base durchgeführt wird.

16. Verfahren zur Markierung eines biologischen Makromoleküls, das die kovalente Konjugation einer Verbindung nach einem oder mehreren der Ansprüche 1 bis 8 an ein biologisches Makromolekül unter Verwendung von R₂ umfaßt, und wahlweise einen Aktivator, um das Produkt an das Makromolekül anzubinden.

17. Verfahren zur Verwendung der Verbindungen nach einem oder mehreren der Ansprüche 1 bis 8, das folgende Schritte umfaßt:
a) Kupplung der Verbindung an ein biologisches Makromolekül, das bei der interessierenden Reaktion verwendet werden soll; und
b) Bestimmen der Menge eines solchen Makromoleküls durch Messen der Fluoreszenz der Verbindung.

18. Konjugat, das eine Verbindung nach einem oder mehreren der Ansprüche 1 bis 8 umfaßt, worin R₂ mit einem biologischen Makromolekül gekuppelt ist.

19. Erfindung nach einem oder mehreren der Ansprüche 16 bis 18, worin das biologische Makromolekül ein Glied eines spezifischen Bindungspaares ist.

20. Erfindung nach Anspruch 19, worin das Glied eines spezifischen Bindungspaares aus einem Antikörper, einem Antigen und einem Hapten gewählt ist.

21. Erfindung nach Anspruch 19, worin das Glied eines spezifischen Bindungspaares ein Oligonucleotid ist.

## Patentansprüche (Patentansprüche für folgende(n) Vertragsstaat(en): ES)

1. Verfahren zur Herstellung einer Verbindung nach folgender Formel: 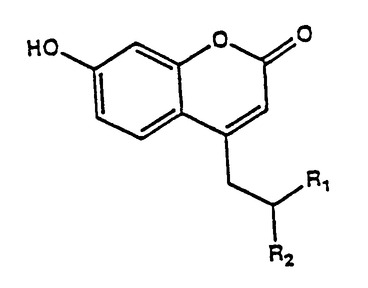 worin R₁ aus H, -CH₃, -G-OZ, -G-SZ, G-NHY, -SZ, -NHY, substituiertem Phenyl und aus substituiertem oder unsubstituiertem Alkyl der allgemeinen Formel -G-CH₃ gewählt ist, wobei G eine Alkylenkette mit 1 bis 25 Kohlenstoffatomen darstellt, und wobei Z eine Schutzgruppe für O oder S darstellt, und wobei Y eine Schutzgruppe für N darstellt; und
worin R₂ aus -J-OH, -J-SH, -J-NH₂, -J-OTs, -J-X, -SH, -NH₂ und -COOR' gewählt ist, worin J eine Alkylenkette mit 1 bis 10 Kohlenstoffatomen darstellt, worin X ein Halogen darstellt, und worin R' Wasserstoff oder eine Alkylkette mit 1 bis 10 Kohlenstoffatomen darstellt, vorausgesetzt, daß wenn R₁ gleich - CH₃ ist, dann R₂ gleich -COOH oder -COOCH₂CH₃ ist;
wobei das Verfahren folgende Schritte umfaßt:
a) Umsetzen von 4-Brommethyl-7-methoxycumarin mit einem Malonester unter Bedingungen, die zur Durchführung der Esterkondensation ausreichend sind unter Erzeugung des (2-Bis(carbalkoxy)-2-R₁-1-ethyl)cumarinderivats;
b) Entfernen einer der Carbalkoxygruppen aus dem Produkt nach Schritt a);
c) Demethylieren des Produktes nach Schritt b) unter Erzeugung der 7-Hydroxycumarinverbindung; und
d) chemisches Abwandeln des verbleibenden Carbalkoxyesters unter Erzeugung eines gewünschten R₂.

2. Verfahren nach Anspruch 1, worin Schritt d) das Abwandeln des verbleibenden Esters zu seiner Überführung in ein Glied umfaßt, das aus -J-OH, -J-SH, -J-NH₂, -J-OTs, -J-X, -SH, -NH₂ und -COOR' gewählt ist, worin J eine Alkylenkette mit 1 bis 5 Kohlenstoffatomen darstellt, worin X ein Halogen darstellt, und worin R' Wasserstoff oder eine Alkylkette mit 1 bis 10 Kohlenstoffatomen darstellt.

3. Verfahren nach Anspruch 1 oder 2, worin Schritt d) die Verseifung des verbleibenden Esters zu einer Carbonsäure umfaßt.

4. Verfahren nach Anspruch 1 oder 2, worin Schritt d) das Abwandeln des verbleibenden Esters zu einer Aminogruppe umfaßt.

5. Verfahren nach Anspruch 1 oder 2, worin Schritt d) das Abwandeln des verbleibenden Esters zu einer Hydroxylgruppe umfaßt.

6. Verfahren nach einem oder mehreren der Ansprüche 1 bis 5, worin Schritt b) in Gegenwart von NaCl und DMSO bei hohen Temperaturen durchgeführt wird.

7. Verfahren nach einem oder mehreren der Ansprüche 1 bis 6, worin Schritt c) in Gegenwart einer starken Lewis-Säure und einer schwachen nukleophilen Lewis-Base durchgeführt wird.

8. Verfahren zur Markierung eines biologischen Makromoleküls, das die kovalente Konjugation des Produktes nach Schritt d) nach einem oder mehreren der Ansprüche 1 bis 7 an ein biologisches Makromolekül unter Verwendung von R₂ umfaßt, und wahlweise einen Aktivator, um das Produkt an das Makromolekül anzubinden.

9. Verfahren zur Verwendung einer Verbindung nach folgender Formel: 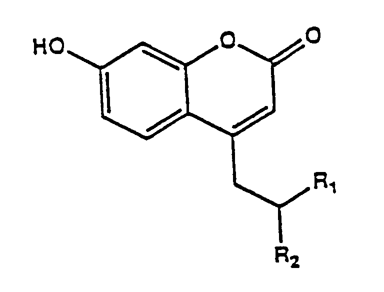 worin R₁ aus H, -CH₃, -G-OZ, -G-SZ, G-NHY, -SZ, -NHY, substituiertem Phenyl und aus substituiertem oder unsubstituiertem Alkyl der allgemeinen Formel -G-CH₃ gewählt ist, wobei G eine Alkylenkette mit 1 bis 25 Kohlenstoffatomen darstellt, und wobei Z eine Schutzgruppe für O oder S darstellt, und wobei Y eine Schutzgruppe für N darstellt; und
worin R₂ aus -J-OH, -J-SH, -J-NH₂_{,} -J-OTs, -J-X, -SH, -NH₂ und -COOR' gewählt ist, worin J eine Alkylenkette mit 1 bis 10 Kohlenstoffatomen darstellt, worin X ein Halogen darstellt, und worin R' Wasserstoff oder eine Alkylkette mit 1 bis 10 Kohlenstoffatomen darstellt, vorausgesetzt, daß wenn R₁ gleich - CH₃ ist, dann R₂ gleich -COOH oder -COOCH₂CH₃ ist;
wobei das Verfahren folgende Schritte umfaßt:
a) Kupplung der Verbindung an ein biologisches Makromolekül, das bei der interessierenden Reaktion verwendet werden soll; und
b) Bestimmen der Menge eines solchen Makromoleküls durch Messen der Fluoreszenz der Verbindung.

10. Verfahren nach Anspruch 9, worin Z aus t-Butyldimethylsilyl, Tetrahydropyran und Triphenylmethyl gewählt ist.

11. Verfahren nach einem oder mehreren der Ansprüche 9 oder 10, worin Y gleich Acetyl ist.

12. Verfahren nach einem oder mehreren der Ansprüche 9 bis 11, worin R₁ gleich Alkyl ist und G eine Alkylenkette mit 1 bis 10 Kohlenstoffatomen ist.

13. Verfahren nach einem oder mehreren der Ansprüche 9 bis 12, worin J eine Alkylenkette mit 1 bis 5 Kohlenstoffatomen ist.

14. Verfahren nach Anspruch 9, worin R₂ gleich -COOH ist.

15. Verfahren nach Anspruch 9 oder 12, worin R₁ aus Methyl, Ethyl und Isopropyl ausgewählt ist.

16. Verbindung nach einem oder mehreren der Ansprüche 9 bis 12, worin R₂ gleich -COOR' ist, wobei R' Wasserstoff oder eine Alkylkette mit 1 bis 10 Kohlenstoffatomen darstellt.

17. Erfindung nach einem oder mehreren der Ansprüche 8 oder 9, worin das biologische Makromolekül ein Glied eines spezifischen Bindungspaares ist.

18. Erfindung nach Anspruch 17, worin das Glied eines spezifischen Bindungspaares aus einem Antikörper, einem Antigen und einem Hapten gewählt ist.

19. Erfindung nach Anspruch 17, worin das Glied eines spezifischen Bindungspaares ein Oligonucleotid ist.

## Revendications (Revendications pour l'(les) Etat(s) contractant(s) suivant(s): DE, FR, IT)

1. Composé de formule : 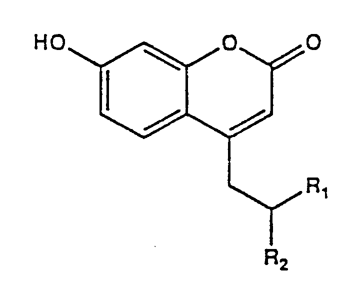 dans laquelle R₁ est choisi parmi H, -CH₃, -G-OZ, -G-SZ, -G-NHY, -SZ, -NHY, un groupement phényle substitué et un groupement alkyle substitué ou non substitué de formule -G-CH₃, G représentant une chaîne alkylène ayant de 1 à 25 atomes de carbone, Z représentant un groupement protecteur pour O ou S et Y représentant un groupement protecteur pour N; et
dans laquelle R₂ est choisi parmi -J-OH, -J-SH, -J-NH₂, -J-OTs, -J-X, -SH, -NH₂ et -COOR', J représentant une chaîne alkylène ayant de 1 à 10 atomes de carbone, X représentant un atome d'halogène et R' représentant l'atome d'hydrogène ou une chaîne alkyle ayant de 1 à 10 atomes de carbone, à condition que lorsque R₁ représente -CH₃, R₂ représente -COOH ou -COOCH₂CH₃.

2. Composé selon la revendication 1, pour lequel Z est choisi parmi le groupement t-butyldiméthylsilyle, le résidu tétrahydropyrane et le groupement triphénylméthyle.

3. Composé selon la revendication 1 ou 2, pour lequel Y représente le groupement acétyle.

4. Composé selon l'une quelconque des revendications 1 à 3, pour lequel R₁ représente un groupement alkyle et G représente une chaîne alkylène ayant de 1 à 10 atomes de carbone.

5. Composé selon l'une quelconque des revendications 1 à 4, pour lequel J représente une chaîne alkylène ayant de 1 à 5 atomes de carbone.

6. Composé selon la revendication 1, pour lequel R₂ représente -COOH.

7. Composé selon la revendication 1 ou 4, pour lequel R₁ est choisi parmi le groupement méthyle, le groupement éthyle et le groupement isopropyle.

8. Composé selon l'une quelconque des revendications 1 à 4, pour lequel R₂ représente -COOR', R' représentant l'atome d'hydrogène ou une chaîne alkyle ayant de 1 à 10 atomes de carbone.

9. Procédé de préparation d'un composé de formule : 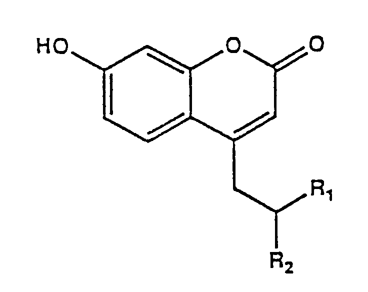 dans laquelle R₁ est choisi parmi H, -CH₃, -G-OZ, -G-SZ, -G-NHY, -SZ, -NHY, un groupement phényle substitué et un groupement alkyle substitué ou non substitué de formule générale -G-CH₃, G représentant une chaîne alkylène ayant de 1 à 25 atomes de carbone, Z représentant un groupement protecteur pour O ou S et Y représentant un groupement protecteur pour N; et
dans laquelle R₂ est choisi parmi -J-OH, -J-SH, -J-NH₂, -J-OTs, -J-X, -SH, -NH₂ et -COOR', J représentant une chaîne alkylène ayant de 1 à 10 atomes de carbone, X représentant un atome d'halogène et R' représentant l'atome d'hydrogène ou une chaîne alkyle ayant de 1 à 10 atomes de carbone à condition que lorsque R₁ représente -CH₃, R₂ représente -COOH ou -COOCH₂CH₃,
ledit procédé comprenant les étapes consistant à :
a) mettre à réagir de la 4-bromométhyl-7-méthoxycoumarine avec un ester malonique dans des conditions suffisantes pour avoir la condensation de l'ester pour obtenir le dérivé (2-bis(carbalcoxy)-2-R₁-1-éthyl)coumarine;
b) éliminer un des groupements carbalcoxy du produit de l'étape a);
c) déméthyler le produit de l'étape b) pour obtenir le composé 7-hydroxycoumarine ; et
d) modifier chimiquement l'ester à groupement carbalcoxy restant pour obtenir un R₂ souhaité.

10. Procédé selon la revendication 9, dans lequel l'étape d) comprend la modification de l'ester restant pour le transformer en un élément choisi parmi -J-OH, -J-SH, -J-NH₂, -J-OTs, -J-X, -SH, -NH₂ et -COOR', J représentant une chaîne alkylène ayant de 1 à 5 atomes de carbone X représentant un atome d'halogène et R' représentant l'atome d'hydrogène ou une chaîne alkyle ayant de 1 à 10 atomes de carbone.

11. Procédé selon la revendication 9 ou 10, dans lequel l'étape d) comprend la saponification de l'ester restant en un acide carboxylique.

12. Procédé selon la revendication 9 ou 10, dans lequel l'étape d) comprend la modification de l'ester restant en un groupement amino.

13. Procédé selon la revendication 9 ou 10, dans lequel l'étape d) comprend la modification de l'ester restant en un groupement hydroxyle.

14. Procédé selon l'une quelconque des revendications 9 à 13, dans lequel l'étape b) est réalisée en présence de NaCl et de DMSO à des températures élevées.

15. Procédé selon l'une quelconque des revendications 9 à 14, dans lequel l'étape c) est réalisée en présence d'un acide fort de Lewis et d'une base nucléophile faible de Lewis.

16. Procédé de marquage d'une macromolécule biologique comprenant la conjugaison par liaison covalente d'un composé selon l'une quelconque des revendications 1 à 8 à une macromolécule biologique en utilisant R₂ et éventuellement un activateur pour lier ledit produit à ladite macromolécule.

17. Procédé d'utilisation de composés selon l'une quelconque des revendications 1 à 8, comprenant les étapes consistant à :
a) coupler le composé à une macromolécule biologique à utiliser dans une réaction intéressante ; et
b) déterminer la quantité de cette macromolécule par la mesure de la fluorescence du composé.

18. Produit conjugué comprenant un composé selon l'une quelconque des revendications 1 à 8, pour lequel R₂ est couplé à une macromolécule biologique.

19. Invention selon l'une quelconque des revendications 16 à 18, dans laquelle ladite macromolécule biologique est un élément d'une paire de liaison spécifique.

20. Invention selon la revendication 19, dans laquelle ledit élément d'une paire de liaison spécifique est choisi parmi un anticorps, un antigène et un haptène.

21. Invention selon la revendication 19, dans laquelle ledit élément d'une paire de liaison spécifique est un oligonucléotide.

## Revendications (Revendications pour l'(les) Etat(s) contractant(s) suivant(s): ES)

1. Procédé de préparation d'un composé de formule : 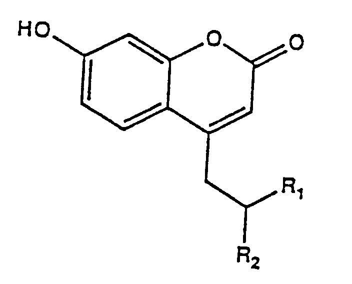 dans laquelle R₁ est choisi parmi H, -CH₃, -G-OZ, -G-SZ, -G-NHY, -SZ, -NHY, un groupement phényle substitué et un groupement alkyle substitué ou non substitué de formule générale -G-CH₃, G représentant une chaîne alkylène ayant de 1 à 25 atomes de carbone, Z représentant un groupement protecteur pour O ou S et Y représentant un groupement protecteur pour N ; et
dans laquelle R₂ est choisi parmi -J-OH, -J-SH, -J-NH₂, -J-OTs, -J-X, -SH, -NH₂ et -COOR', J représentant une chaîne alkylène ayant de 1 à 10 atomes de carbone, X représentant un atome d'halogène et R' représentant l'atome d'hydrogène ou une chaîne alkyle ayant de 1 à 10 atomes de carbone, à condition que lorsque R₁ représente -CH₃, R₂ représente -COOH ou -COOCH₂CH₃,
ledit procédé comprenant les étapes consistant à :
a) mettre à réagir de la 4-bromométhyl-7-méthoxycoumarine avec un ester malonique dans des conditions suffisantes pour avoir la condensation de l'ester pour obtenir le dérivé (2-bis(carbalcoxy)-2-R₁-1-éthyl)coumarine;
b) éliminer un des groupements carbalcoxy du produit de l'étape a);
c) déméthyler le produit de l'étape b) pour obtenir le composé 7-hydroxycoumarine; et
d) modifier chimiquement l'ester à groupement carbalcoxy restant pour obtenir un R₂ souhaité.

2. Procédé selon la revendication 1, dans lequel l'étape d) comprend la modification de l'ester restant pour le transformer en un élément choisi parmi -J-OH, -J-SH, -J-NH₂, -J-OTs, -J-X, -SH, -NH₂ et -COOR', J représentant une chaîne alkylène ayant de 1 à 5 atomes de carbone, X représentant un atome d'halogène et R' représentant l'atome d'hydrogène ou une chaîne alkyle ayant de 1 à 10 atomes de carbone.

3. Procédé selon la revendication 1 ou 2, dans lequel l'étape d) comprend la saponification de l'ester restant en un acide carboxylique.

4. Procédé selon la revendication 1 ou 2, dans lequel l'étape d) comprend la modification de l'ester restant en un groupement amino.

5. Procédé selon la revendication 1 ou 2, dans lequel l'étape d) comprend la modification de l'ester restant en un groupement hydroxyle.

6. Procédé selon l'une quelconque des revendications 1 à 5, dans lequel l'étape b) est réalisée en présence de NaCl et de DMSO à des températures élevées.

7. Procédé selon l'une quelconque des revendications 1 à 6, dans lequel l'étape c) est réalisée en présence d'un acide fort de Lewis et d'une base nucléophile faible de Lewis

8. Procédé de marquage d'une macromolécule biologique comprenant la conjugaison par liaison covalente du produit de l'étape d) selon l'une quelconque des revendications 1 à 7 à une macromolécule biologique en utilisant R₂ et éventuellement un activateur pour lier ledit produit à ladite macromolécule.

9. Procédé d'utilisation d'un composé de formule : 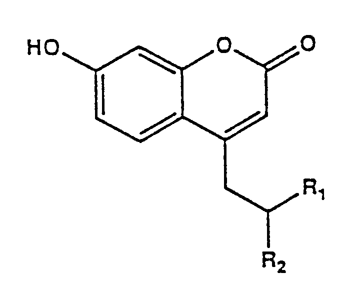 dans laquelle R₁ est choisi parmi H, -CH₃, -G-OZ, -G-SZ, -G-NHY, -SZ, -NHY, un groupement phényle substitué et un groupement alkyle substitué ou non substitué de formule -G-CH₃, G représentant une chaîne alkylène ayant de 1 à 25 atomes de carbone, Z représentant un groupement protecteur pour O ou S et Y représentant un groupement protecteur pour N ; et
dans laquelle R₂ est choisi parmi-J-OH, -J-SH, -J-NH₂, -J-OTs, -J-X, -SH, -NH₂ et -COOR', J représentant une chaîne alkylène ayant de 1 à 10 atomes de carbone, X représentant un atome d'halogène et R' représentant l'atome d'hydrogène ou une chaîne alkyle ayant de 1 à 10 atomes de carbone, à condition que lorsque R₁ représente -CH₃, R₂ représente -COOH ou -COOCH₂CH₃,
ledit procédé comprenant les étapes consistant à :
a) coupler le composé à une macromolécule biologique à utiliser dans une réaction intéressante ; et
b) déterminer la quantité de cette macromolécule par la mesure de la fluorescence du composé.

10. Procédé selon la revendication 9, pour lequel Z est choisi parmi le groupement t-butyldiméthylsilyle, le résidu tétrahydropyrane et le groupement triphénylméthyle.

11. Procédé selon la revendication 9 ou 10, pour lequel Y représente le groupement acétyle.

12. Procédé selon l'une quelconque des revendications 9 à 11, pour lequel R₁ représente un groupement alkyle et G représente une chaîne alkylène ayant de 1 à 10 atomes de carbone.

13. Procédé selon l'une quelconque des revendications 9 à 12, pour lequel J représente une chaîne alkylène ayant de 1 à 5 atomes de carbone.

14. Procédé selon la revendication 9, pour lequel R₂ représente -COOH.

15. Procédé selon la revendication 9 ou 12, pour lequel R₁ est choisi parmi le groupement méthyle, le groupement éthyle et le groupement isopropyle.

16. Procédé selon l'une quelconque des revendications 9 à 12, pour lequel R₂ représente -COOR', R' représentant l'atome d'hydrogène ou une chaîne alkyle ayant de 1 à 10 atomes de carbone.

17. Invention selon la revendication 8 ou 9, dans laquelle ladite macromolécule biologique est un élément d'une paire de liaison spécifique.

18. Invention selon la revendication 17, dans laquelle ledit élément d'une paire de liaison spécifique est choisi parmi un anticorps, un antigène et un haptène.

19. Invention selon la revendication 17, dans laquelle ledit élément d'une paire de liaison spécifique est un oligonucléotide.
